# EUROPEAN PATENT APPLICATION

(11) **EP 3 037 100 A1**
(43) Date of publication of application: **29.06.2016**
(21) Application number: 14774469.2
(22) Date of filing: 25.03.2014
(51) Int. Cl.: A61K 38/00, A23K 10/00, A23K 10/28, A23K 10/40, A23L 33/17, A61K 35/20, A61P 21/06

(54) **MUSCLE-ATROPHY-PREVENTING AGENT**

(30) Priority: 28.03.2013 JP 2013070414
(71) Applicant: Megmilk Snow Brand Co. Ltd., Sapporo-shi, Hokkaido 065-0043 (JP)
(72) Inventor: ISHIDA, Yuko, Sapporo-shi Hokkaido 065-0043 (JP); MORITA, Yoshikazu, Sapporo-shi Hokkaido 065-0043 (JP); KATOH, Ken, Sapporo-shi Hokkaido 065-0043 (JP); KOBAYASHI, Toshiya, Sapporo-shi Hokkaido 065-0043 (JP); MIZUNO, Yuki, Sapporo-shi Hokkaido 065-0043 (JP); OHMACHI, Aiko, Sapporo-shi Hokkaido 065-0043 (JP)
(74) Representative: Høiberg A/S
(86) International application number: PCT/JP2014/058205
(87) International publication number: WO 2014/157153

(57) **Abstract**

A muscle atrophy-preventing agent includes a milk-derived basic protein fraction and/or a decomposed product of the milk-derived basic protein fraction as an active ingredient. The muscle atrophy-preventing agent is remarkably effective in preventing muscle mass decline and increasing muscle mass, and thus is useful in preventing and treating various muscle diseases, such as sarcopenia and disuse muscle atrophy.

## Description

### TECHNICAL FIELD

The present invention relates to a muscle atrophy-preventing agent that prevents muscle atrophy in vivo and is effective in prevention and treatment of various muscle diseases, such as sarcopenia and disuse muscle atrophy. The present invention also relates to a food or drink for preventing muscle atrophy, a nutritional composition for preventing muscle atrophy, and a feed for preventing muscle atrophy, which contain the muscle atrophy-preventing agent.

### BACKGROUND ART

In recent years, Japan has reached a super-aged society in which the average life expectancy is approaching 80 years and about one in four persons is aged 65 or over. Along with this situation, the prevalence of functional deterioration in the locomotive organs has also been continuing to increase. The Japanese Orthopaedic Association thus proposed a new term "locomotive syndrome" in 2007 to promote changes in the awareness of the public and physicians about the health maintenance and health promotion of the locomotor system and the nursing care. The locomotive syndrome refers to a health condition requiring actual nursing care or having a high risk of nursing care due to locomotor dysfunction. The locomotor system includes in general organs to support and move the body, such as bones, joints, and ligaments; spine and spinal cord; muscles and tendons, and peripheral nerves. Typical examples of the diseases and dysfunctions in the locomotor system include osteoporosis, sarcopenia, and osteoarthritis.

Sarcopenia refers to a syndrome characterized by a progressive and systemic decline in skeletal muscle mass and strength. Sarcopenia is defined as a decline in muscle mass and strength occurring in relation to aging in a narrow sense, while it refers to a decline in muscle mass and strength due to various causes in a broad sense. Sarcopenia in a broad sense is categorized into a primary (age-related) sarcopenia that has no apparent cause other than aging and a secondary sarcopenia that has one or more apparent causes other than aging. The secondary sarcopenia is divided into three main groups of those caused by a decline in activity level, a disease, and an insufficient intake of nutrients. In other words, patients with the secondary sarcopenia are not limited to the elderly. For example, a disuse muscle atrophy that can occur when muscles have not been used for an extended period of time due to, e.g. , hospitalization, is also categorized into one of the secondary sarcopenia.

The onset and progression of sarcopenia are associated with the metabolism of skeletal muscle protein. The amount of skeletal muscles is adjusted by the balance between muscle protein synthesis and degradation. Imbalance therebetween, i.e., synthesis falling below degradation or degradation exceeding synthesis, causes muscle atrophy, resulting in a decline in the amount of muscles. In the elderly, muscle atrophy is caused by a decline in the secretion of growth hormone that promotes protein synthesis and inhibits protein degradation or an increase in the level in the blood of adrenal cortex hormone (glucocorticoid) that inhibits protein synthesis, resulting in synthesis lower than degradation. Meanwhile, in the secondary sarcopenia, muscle atrophy is caused by a decline in protein synthesis due to lack of locomotor stimulation to skeletal muscles, resulting in degradation greater than synthesis.

To prevent and ameliorate muscle diseases, such as sarcopenia in a physical way, high-intensity resistance training is proved to be effective. To actively carry out such high-intensity resistance training, however, imposes a large physical burden to elderly and convalescent patients and also requires appropriate guidance by experts. For this reason, nutritional approaches to prevention and amelioration of sarcopenia, which can readily be provided to even a person whose basic physical strength or motor functioning is declined, have been awaited.

Examples of food components having an effect in improving the muscle function, which are effective to prevent and ameliorate muscular disorders, such as sarcopenia, disclosed include fruit polyphenols inhibiting muscle atrophy (Patent Document 1); lycopene inhibiting protein degradation (Patent Document 2); and catechins inhibiting muscle aging (Patent Document 3). Moreover, research has been conducted on prevention of and early recovery from skeletal muscle atrophy by ingestion of proteins and amino acids. In particular, whey protein contained in milk, which is known to have a higher content of branched-chain amino acids (BCAA; involved in synthesis of muscle) compared with soy protein, and also high net protein utilization (NPU), has been utilized as a supplement for muscle enhancement and recovery during exercise. Unfortunately, these components have drawbacks in that daily intake of them can be a burden due to a large amount needed to be effective and that the specific flavors or colors of the components added to food and drink adversely affect the sensory attributes of food and drink.

### RELATED ART

Patent Document 1: Japanese Patent Application Laid-Open No. 2001-89387
Patent Document 2: Japanese Patent Application Laid-Open No. 2004-59518
Patent Document 3: Japanese Patent Application Laid-Open No. 2008-63321

### SUMMARY OF INVENTION

An object of the present invention is to provide a muscle atrophy-preventing agent having an effect of preventing muscle atrophy in vivo; and a food or drink for preventing muscle atrophy, a nutritional composition for preventing muscle atrophy, or a feed for preventing muscle atrophy, which contain the muscle atrophy-preventing agent.

### SOLUTION TO PROBLEM

The present inventors, who have conducted extensive research to solve the above problems, have found that a milk-derived basic protein fraction and its decomposed product have an effect on preventing muscle atrophy, and then have completed the invention.

The present invention includes the following aspects: Aspect (1) a muscle atrophy-preventing agent including a milk-derived basic protein fraction and/or a decomposed product of the milk-derived basic protein fraction as an active ingredient.
Aspect (2) the muscle atrophy-preventing agent according to Aspect (1), wherein the decomposed product of the milk-derived basic protein fraction is prepared by treating the milk-derived basic protein fraction with a protease.
Aspect (3) the muscle atrophy-preventing agent according to Aspect (2), wherein the protease is at least one selected from the group consisting of pepsin, trypsin, chymotrypsin, pancreatin, and papain.
Aspect (4) the muscle atrophy-preventing agent according to any one of Aspects (1) to (3), wherein the milk-derived basic protein fraction has an amino acid composition containing at least 15 weight% basic amino acids.
Aspect (5) the muscle atrophy-preventing agent according to any one of Aspects (1) to (3), wherein the milk-derived basic protein fraction is prepared by bringing milk or a milk-derived raw material into contact with a cation exchange resin to adsorb basic proteins on the resin, and eluting a fraction of the adsorbed basic proteins with an eluent having a salt concentration of 0.1 M to 1.0 M.
Aspect (6) A food or drink for preventing muscle atrophy, a nutritional composition for preventing muscle atrophy, or a feed for preventing muscle atrophy, containing the muscle atrophy-preventing agent according to any one of Aspects (1) to (4).
Aspect (7) A method of preventing muscle atrophy, including: ingestion of a milk-derived basic protein fraction and/or a decomposed product of the milk-derived basic protein fraction in an amount of 5 mg or more per day.
Aspect (8) Use of a muscle atrophy-preventing agent including a milk-derived basic protein fraction and/or a decomposed product of the milk-derived basic protein fraction as an active ingredient applied to patients with muscle diseases. Aspect (9) Use of a milk-derived basic protein fraction and/or a decomposed product of the milk-derived basic protein fraction in production of a muscle atrophy-preventing agent.

### EFFECTS OF INVENTION

The muscle atrophy-preventing agent of the present invention can prevent muscle atrophy in vivo and is effective in prevention and treatment of various muscle diseases, such as sarcopenia and disuse muscle atrophy.

### DESCRIPTION OF EMBODIMENT

In an embodiment, the muscle atrophy-preventing agent includes a milk-derived basic protein fraction and/or a decomposed product of the milk-derived basic protein fraction as an active ingredient. The milk-derived basic protein fraction can be prepared from the milk of a mammal, such as a cow, human, goat, and sheep and can also be treated with a protease to yield a decomposed product of the milk-derived basic protein fraction.

The milk-derived basic protein fraction preferably has the following characteristics:
1) The fraction contains several proteins having molecular weights in a range of 3,000 to 80,000 in accordance with sodium dodecyl sulfate-polyacrylamide gel electrophoresis (SDS-PAGE);
2) The fraction contains 95 weight% or more proteins and small amounts of fats and ashes;
3) The major proteins are lactoferrin and lactoperoxidase; and
4) The proteins each have an amino acid composition containing at least 15 weight% basic amino acids, such as lysine, histidine, and arginine.

The milk-derived basic protein fraction can be prepared, for example, by bringing a milk-derived raw material, such as skimmed milk and whey, into contact with a cation exchange resin to adsorb basic proteins on the resin; eluting a fraction of the adsorbed basic proteins with an eluent having a salt concentration of 0.1 M to 1. 0 M; collecting the eluted fraction to desalt and concentrate through a membrane process, such as reverse osmosis (RO) and electrodialysis (ED), and optionally drying the resultant. Other known examples of preparing a milk-derived basic protein fraction include a procedure of bringing milk or a milk-derived raw material in contact with a cation exchanger to adsorb basic proteins on the exchanger and then eluting a fraction of the basic proteins with an eluent having a pH of higher than 5 and an ionic strength of higher than 0.5 (Japanese Patent Application Laid-Open No. H05-202098) ; a procedure using an alginate gel (Japanese Patent Application Laid-Open No. S61-246198); a procedure of preparing from whey using inorganic porous particles (Japanese Patent Application Laid-Open No. H01-86839), and a procedure of preparing from milk using a sulfated ester compound (Japanese Patent Application Laid-Open No. S63-255300). The milk-derived basic protein fraction prepared through any of these procedures can be used. In particular, preferably used is a milk-derived basic protein fraction prepared by bringing milk or a milk-derived raw material into contact with a cation exchange resin to adsorb basic proteins on the resin and then eluting a fraction of the adsorbed basic proteins with an eluent having a salt concentration of 0.1 M to 1.0 M. Furthermore, a decomposed product of the milk-derived basic protein fraction has the same amino acid composition as that of the milk-derived basic protein fraction and thus the milk-derived basic protein fraction resulted from any of the above procedures can be treated with a protease(s) to obtain a decomposed product of the milk-derived basic protein fraction having an average molecular weight of 4,000 or less in accordance with sodium dodecyl sulfate-polyacrylamide gel electrophoresis (SDS-PAGE). It should be noted that usable examples of such a protease include commercially available proteases for food or industrial uses, such as Protease A "Amano" SD (brand name), Thermoase PC10F (brand name), and Protin SD-AY10 (brand name); and also enzymes, such as pepsin, trypsin, chymotrypsin, pancreatin, and papain. These proteases may also be used appropriately in combination.

The milk-derived basic protein fraction and/or the decomposed product of the milk-derived basic protein fraction prepared through any procedure described above can directly be used as a muscle atrophy-preventing agent or can be used in a form of powder, granule, tablet, capsule, or health drink formulated in a usual manner as necessary.

The milk-derived basic protein fraction and/or the decomposed product of the milk-derived basic protein fraction can be formulated into a drug or added/blended to, e.g., foods and drinks, through any procedure. For example, to add/blend in a solution, the milk-derived basic protein fraction and/or the decomposed product of the milk-derived basic protein fraction may be suspended or dissolved in deionized water, mixed with stirring, and then formulated into a drug or prepared into a food or drink, or feed for use.

The mixing with stirring may be performed under any condition for homogenous mixing of the milk-derived basic protein fraction and/or the decomposed product of the milk-derived basic protein fraction. For example, an ultra disperser or TK homomixer may be used to mix with stirring. Furthermore, the solution may be concentrated with, e.g., an RO membrane, or lyophilized as necessary to be readily formulated into a drug, food or drink, or feed. In the present invention, sterilization treatment that is normally employed in production of pharmaceuticals, foods or drinks, or feeds can be performed. In a powder form, dry heat sterilization can also be performed.

The muscle atrophy-preventing agent can thus be formed into various shapes, such as liquid, gel, powder, and granules. Furthermore, the formulated muscle atrophy-preventing agent can also be blended to nutrients; foods or drinks, such as yogurt, milk beverages, and wafers; and nutritional compositions.

The muscle atrophy-preventing agent contains a milk-derived basic protein fraction and/or a decomposed product of the milk-derived basic protein fraction as an active ingredient. The formulated agent may also contain diluents or excipients, such as fillers, bulking fillers, binders, disintegrants, surfactants, and lubricants that are commonly used in formulation. Examples of excipients include sucrose, lactose, starch, microcrystalline cellulose, mannitol, light anhydrous silicate, magnesium aluminate, synthetic aluminum silicate, magnesium aluminometasilicate, calcium carbonate, sodium hydrogen carbonate, calcium hydrogen phosphate, and carboxymethylcellulose calcium, which can be added alone or in combination.

The muscle atrophy-preventing agent may also contain stabilizers, sugars, fats, flavors, vitamins, minerals, flavonoids, and/or polyphenols in any combination, which may be appropriately blended to prepare a food or drink, or feed. Other components, which exhibit an effect of improving muscle function, such as fruit polyphenols, lycopene, catechins, branched-chain amino acids (BCAA), and soy proteins may also be used together.

The muscle atrophy-preventing agent can prevent muscle atrophy in vivo by orally administering 5 mg or more of the agent per kg body weight to mice and rats as shown in the testing examples described later. Since the intake in experimental animals is equivalent to the intake per adult in terms of blood drug concentration (Mitsuyoshi Nakashima (1993), "Evaluation of medicinal efficacy (Yakko Hyoka), Vol. 8", Hirokawa Publishing Company, pp. 2-18), ingestion of 5 mg or more of the muscle atrophy-preventing agent per adult per day in general can be expected to have an effect in preventing and treating muscle atrophy in vivo, particularly sarcopenia and disuse muscle atrophy. The milk-derived basic protein fraction and/or the decomposed product of the milk-derived basic protein fraction may thus be formulated into a drug, or blended to foods or drinks in an amount that ensures intake of the required amount. For example, to ingest 5 mg or more of the milk-derived basic protein fraction and/or the decomposed product of the milk-derived basic protein fraction per adult per day, the amount of the milk-derived basic protein fraction and/or the decomposed product of the milk-derived basic protein fraction contained in a final product may be 0.05 to 200 mg per 100 g of pharmaceuticals, foods or drinks, or feeds although it depends on their forms.

Although the present invention has been described based on the foregoing embodiment, the description that forms a part of this disclosure should not be construed to limit the present invention. From this disclosure, various alternative embodiments, examples and operational techniques will be apparent to those skilled in the art.

For example, the muscle atrophy-preventing agent comprising the milk-derived basic protein fraction and/or the decomposed product of the milk-derived basic protein fraction as its active ingredient described in the embodiment can be provided for use of applying the agent to patients with various muscle diseases, such as sarcopenia and disuse muscle atrophy. The muscle atrophy-preventing agent may be applied to humans and also to other mammals, for examples, domestic animals, such as dogs, monkeys, cats, cattle, horses, pigs, chickens, and sheep.

Use of the milk-derived basic protein fraction and/or the decomposed product of the milk-derived basic protein fraction in production of the muscle atrophy-preventing agent can also be provided.

### EXAMPLES

The present invention will be described in detail by way of examples and testing examples that are provided only by way of illustration. The invention should not be limited to these examples.

### [Example 1]

A column (5 cm in diameter x 30 cm in height) filled with 400 g of sulfonated Chitopearl (a cation exchange resin, available from Fuji Spinning Co., Ltd.) was thoroughly washed with deionized water and then 40 L of unsterilized skimmed milk (pH 6.7) was passed through the column at a flow rate of 25 mL/min. After the milk was passed through, the column was thoroughly washed with deionized water and then a basic protein fraction adsorbed on the resin was eluted with a 0.02 M carbonate buffer solution (pH 7.0) containing 0.98 M sodium chloride. This procedure was repeated 10 times. The eluted solution was desalted and concentrated through a reverse osmosis (RO) membrane, and then lyophilized to obtain 210 g of powdery milk-derived basic protein fraction (Example Product 1). The resulting milk-derived basic protein fraction was determined with sodium dodecyl sulfate-polyacrylamide gel electrophoresis (SDS-PAGE) to have a molecular weight distribution in a range of 3,000 to 80,000 and a component composition as shown in Table 1. The fraction was also hydrolyzed with 6N hydrochloric acid at 110 °C for 24 hours and then its amino acid composition was analyzed with an amino acid analyzer (model L-8500, available from Hitachi, Ltd.). As shown in Table 2, the total content of the basic amino acids exceeds 15 weight%. The protein composition of the fraction was further analyzed by ELISA. As shown in Table 3, the lactoferrin and lactoperoxidase contents exceed 40%.

**[Table 1]**

| | |
|---|---|
| Water | 1.06 (wt%) |
| Proteins | 96.50 |
| Fats | 0.56 |
| Ashes | 0.27 |
| Others | 1.61 |

**[Table 2]**

| | |
|---|---|
| Aspartic acid | 10.1 (wt%) |
| Serine | 5.3 |
| Glutamic acid | 12.3 |
| Proline | 4.7 |
| Alanine | 5.7 |
| Valine | 5.1 |
| Isoleucine | 3.1 |
| Leucine | 10.2 |
| Lysine | 8.4 |
| Histidine | 2.5 |
| Arginine | 7.2 |
| Others | 25.4 |

**[Table 3]**

| | |
|---|---|
| Lactoferrin | 42.5 (wt%) |
| Lactoperoxidase | 45.6 |
| Insulin-like growth factor-1 | 0.005 |
| Others | 11.895 |

### [Example 2]

A column (100 cm in diameter x 10 cm in height) filled with 30 kg of SP TOYOPEARL (a cation exchange resin, available from Tosoh Corporation) was thoroughly washed with deionized water and then 3 t of cheese whey (pH 6.2), which was heat-sterilized at 121°C for 30 seconds, was passed through the column at a flow rate of 10 L/min. After the whey was passed through, the column was thoroughly washed with deionized water and then a basic protein fraction adsorbed on the resin was eluted with a 0.1 M citrate buffer solution (pH 5.7) containing 0.9 M sodium chloride. The eluted solution was desalted and concentrated through an electrodialysis (ED) process, and then lyophilized to obtain 183 g of a powdery milk-derived basic protein fraction (Example Product 2).

### [Example 3]

After 50 g of the milk-derived basic protein fraction prepared in Example 1 was dissolved in 10 L of distilled water, 1% pancreatin (available from Sigma) was added thereto for reaction at 37°C for two hours. After the reaction, the reacted solution was heated at 80°C for 10 minutes to inactivate the enzyme and then lyophilized to obtain 48.3 g of a decomposed product of the milk-derived basic protein fraction (Example Product 3).

### [Example 4]

After 120 g of the milk-derived basic protein fraction prepared in Example 2 was dissolved in 1.8 L of purified water, 20 g of Protease A "Amano" SD (available from Amano Enzyme Inc.) was added thereto, which was kept at 45°C, for reaction for two hours. The reacted solution was heated at 80 °C for 10 minutes to inactivate the enzyme and then lyophilized to obtain 95 g of a decomposed product of the milk-derived basic protein fraction (Example Product 4).

### [Testing Example 1]

### (Test for preventing muscle mass decline)

The milk-derived basic protein fraction of Example Product 1 and the decomposed product of the milk-derived basic protein fraction of Example Product 3 were evaluated for the effect of preventing muscle mass decline.

As experimental animals, 20-week-old SAM-P female mice were used. The mice were divided into 6 weight-matched groups (each group: n=7, 29.8 ± 1.9 g): a group administered with physiological saline (control group) ; groups administered with Example Product 1 and Example Product 3, respectively, in an amount of 5 mg to 10 mg per kg mouse body weight (group of Example Product 1 (5 mg/kg) group of Example Product 1 (10 mg/kg), and group of Example Product 3 (5 mg/kg)) ; a group administered with a whey protein isolate (WPI, available from Fonterra Co-Operative Group Ltd.) in an amount of 120 mg per kg mouse body weight (group of WPI (120 mg/kg) ) ; and a group administered with a catechin mixture derived from green tea (available from Nagara Science Co., Ltd.) in an amount of 120 mg per kg mouse body weight (group of catechin mixture (120 mg/kg)). Each test sample was orally administered once a day with a sonde. The mice were raised until 45 weeks old. It should be noted that Example Products 1 and 3, the WPI, and the catechin mixture each were suspended in physiological saline and then orally administered. At the end of the test, the extensor digitorum longus (EDL), tibialis anterior (TA), soleus muscle (SOL), and gastrocnemius (GAS) were excised from the right hind leg of each mouse under anesthesia with pentobarbital sodium (50 mg/kg). Each muscle was weighed to calculate the relative muscle mass to body weight (mg/100 g of body weight). The results are shown in Table 4.

**[Table 4]**

| Experimental groups | EDL | TA | SOL | GAS |
|---|---|---|---|---|
| Control | 32.16±1.52 a | 143.0±6.9 a | 23.33±1.10 a | 341.7+ 7.9 a |
| Example Product 1 (5 mg/kg) | 34.93+1.39. b | 159.5±7.1 b c | 25.48±1.26 b | 391.1±16.0 b |
| Example Product 1 (10 mg/kg) | 37.00±1.63 c | 167.2±9.1 b | 27.05±0.48 c | 408.8±6.9 c |
| Example Product 3 (5 mg/kg) | 34.32±0.85 b | 159.4±8.5 b c | 25.45±0.91 b | 384.3±12.0 b |
| WPI (120 mg/kg) | 32.21±0.63 a | 147.7±4.3 a d | 23.55±0.88 a | 353.7±7.5 a d |
| Catechin mixture (120 mg/kg) | 34.36±1.82 b | 154.4±6.0 c d | 25.33±0.93 b | 367.4±10.1 d |

| | | | | |
|---|---|---|---|---|
| * Values represent the mean values ± standard deviation. * Significant difference was determined by a Tukey-Kramer test. Different alphabets indicate different significant differences (n=7, p < 0.05). | | | | |

The results in Table 4 show that the relative muscle mass to body weight in all of the extensor digitorum longus, tibialis anterior, soleus muscle, and gastrocnemius was significantly higher in the groups administered with the milk-derived basic protein fraction of Example Product 1 in an amount of 5 mg and 10 mg, respectively, per kg mouse body weight, the group administered with the decomposed product of the milk-derived basic protein fraction of Example Product 3 in an amount of 5 mg per kg mouse body weight, and the group administered with the catechin mixture in an amount of 120 mg per kg mouse body weight compared to the control group. Meanwhile, no significant difference was observed in the relative muscle mass to body weight in all of the extensor digitorum longus, tibialis anterior, soleus muscle, and gastrocnemius between the group administered with the WPI in an amount of 120 mg per kg mouse body weight and the control group.

Since the SAM-P mice used in the test were senescence accelerated mice, the mice in the control group exhibited a declined muscle mass due to muscle atrophy with aging. The groups administered with Example Products 1 and 3 and the catechin mixture exhibited a suppressed age-related muscle atrophy, resulting in a low decline in muscle mass, whereas the group administered with the WPI exhibited no effect.

Moreover, the weight of the extensor digitorum longus, tibialis anterior, and soleus muscle in the groups administered with Example Products 1 and 3, respectively, in an amount of 5 mg per kg mouse body weight was very similar to that in the group administered with the catechin mixture in an amount of 120 mg per kg mouse body weight, whereas the weight of the gastrocnemius in the groups administered with Example Products 1 and 3, respectively, in an amount of 5 mg per kg mouse body weight was significantly higher than that in the group administered with the catechin mixture in an amount of 120 mg per kg mouse body weight. The relative muscle mass to body weight in all of the extensor digitorum longus, tibialis anterior, soleus muscle, and gastrocnemius was significantly higher in the group administered with Example Product 1 in an amount of 10 mg per kg mouse body weight than in the group administered with the catechin mixture in an amount of 120 mg per kg mouse body weight.

The milk-derived basic protein fraction and the decomposed product of the milk-derived basic protein fraction of the present invention thus have a superior effect of preventing muscle mass decline compared to the WPI and the catechin mixture. This effect was obviously observed in the dosage of 5 mg or more per kg mouse body weight.

### [Testing Example 2]

### (Test for increasing muscle mass)

The milk-derived basic protein fraction of Example Product 2 and the decomposed product of the milk-derived basic protein fraction of Example Product 4 were evaluated for the effect of increasing muscle mass.

As experimental animals, 6-week-old Wistar female rats were used. The rats were divided into 7 weight-matched groups (each group: n=7, 145.4 ± 6.5 g) : a normal rearing group without tail suspension (normal rearing group); a group administered with physiological saline during recovering from tail suspension (physiological saline group); groups administered with Example Products 2 and 4, respectively, in an amount of 5 mg to 10 mg per kg rat body weight during recovering from tail suspension (group of Example Product 2 (5 mg/kg), group of Example Product 2 (10 mg/kg), and group of Example Product 4 (5 mg/kg)); a group administered with a whey protein isolate (WPI, available from Fonterra Co-Operative Group Ltd.) in an amount of 120 mg per kg rat body weight during recovering from tail suspension (group of WPI (120 mg/kg)); and a group administered with a catechin mixture derived from green tea (available from Nagara Science Co., Ltd.) in an amount of 120 mg per kg rat body weight during recovering from tail suspension (group of catechin mixture (120 mg/kg)). After the grouping, the rats were raised for one week in a manner as follows: the normal rearing group was kept on in a cage for normal rearing and the rats in the remaining six groups were tail-suspended such that no load was applied to their hind legs. After the tail suspension, all the groups were transferred to cages for normal rearing to be provided with a recovery period and orally administered with each of the test samples mentioned above once a day with a sonde for one week. It should be noted that the normal rearing group was orally administered with physiological saline once a day with a sonde for a period corresponding to the recovery period for the groups subjected to tail suspension (one week). Example Products 2 and 4, the WPI, and the catechin mixture each were suspended in physiological saline and then orally administered. At the end of the test, the soleus muscle (SOL) was excised from each rat under anesthesia with pentobarbital sodium (50 mg/kg) and then weighed to calculate the relative muscle mass to body weight (mg/100 g of body weight) . The results are shown in Table 5.

**[Table 5]**

| Experimental groups | Relative SOL mass to body weight |
|---|---|
| Normal rearing | 32.2±0.6 b |
| Physiological saline | 26.0±0.5 a |
| Example Product 2 (5 mg/kg) | 31.5±0.6 b |
| Example Product 2 (10 mg/kg) | 32.0±0.6 b |
| Example Product 4 (5 mg/kg) | 30.0±0.7 c |
| WPI (120 mg/kg) | 26.8±0.7 a |
| Catechin mixture (120 mg/kg) | 29.0±0.7 c |

| | |
|---|---|
| * Values represent the mean values ± standard deviation. * Significant difference was determined by a Tukey-Kramer test. Different alphabets indicate different significant differences (n=7, p < 0.05). | |

The results in Table 5 show that the soleus muscle mass per body weight was significantly declined in the group subjected to tail suspension and administered with physiological saline compared to the normal rearing group. The soleus muscle mass was, however, significantly increased in the groups administered with Example Product 2 in an amount of 5 mg and 10 mg, respectively, per kg rat body weight and the group administered with the decomposed product of the milk-derived basic protein fraction of Example Product 4 in an amount of 5 mg per kg rat body weight compared to the physiological saline group and the increased degree was very similar to that of the normal rearing group. Meanwhile, no difference was observed in the soleus muscle mass per body weight between the group administered with the WPI in an amount of 120 mg per kg rat body weight and the control. Although the soleus muscle mass was also significantly increased in the group administered with the catechin mixture in an amount of 120 mg per kg rat body weight compared to the physiological saline group, such an effect was lower than the effect exhibited in the groups administered with Example Products 2 and 4, respectively, in an amount of 5 mg or 10 mg per kg rat body weight. The milk-derived basic protein fraction and the decomposed product of the milk-derived basic protein fraction of the present invention thus have a superior effect of increasing muscle mass compared to the WPI and the catechin mixture. This effect was obviously observed in the dosage of 5 mg or more per kg rat body weight.

### [Example 5]

### (Preparation of tablets for preventing muscle atrophy)

The ingredients in the respective amounts shown in Table 6 were mixed together and then formed into tablets (1 g each) for preventing muscle atrophy in a usual manner.

**[Table 6]**

| | |
|---|---|
| Crystalline glucose hydrate | 91.0 (wt%) |
| Example Product 1 | 2.5 |
| Mineral mixture | 5.0 |
| Sugar ester | 1.0 |
| Flavor | 0.5 |

### [Example 6]

### (Preparation of nutritional composition for preventing muscle atrophy)

The decomposed product of the milk-derived basic protein fraction of Example Product 3 (25 g) was dissolved in deionized water (4,975g). The solution was heated to 50°C and then mixed with stirring with a T.K. HOMO MIXER (TK ROBO MICS; available from Tokushu Kika Kogyo Co., Ltd.) at 6,000 rpm for 30 minutes to prepare a solution containing the decomposed product of the milk-derived basic protein fraction of Example Product 3 in an amount of 25 g/5 kg. Subsequently, the solution (5.0 kg) was mixed with casein (5.0 kg), soy protein (5.0 kg), fish oil (1.0 kg) , perilla oil (3.0 kg) , dextrin (17.0 kg), a mineral mixture (6.0 kg), a vitamin mixture (1.95 kg), emulsifier (2.0 kg), stabilizer (4.0 kg), and flavor (0.05 kg), and the mixture was placed into 200-mL retort pouches, which were sterilized in a retort sterilizer (first-class pressure vessel, TYPE: RCS-4CRTGN, available from HISAKA WORKS, LTD.) at 121°C for 20 minutes to produce 50 kg of a liquid nutritional composition for preventing muscle atrophy of the present invention. The liquid nutritional composition for preventing muscle atrophy contained 50 mg of the decomposed product of the milk-derived basic protein fraction of Example product 3 per 100 g of the composition.

### [Example 7]

### (Preparation of drink for preventing muscle atrophy)

Skimmed milk powder (300 g) was dissolved in deionized water (409.5 g), and then the milk-derived basic protein fraction of Example Product 2 (0.5 g) was dissolved therein. The solution was heated to 50°C and then agitated with an ultra disperser (ULTRA-TURRAX T-25; available from IKA Japan) at 9,500 rpm for 30 minutes. Maltitol (100 g), acidulant (2 g), reduced starch syrup (20 g), flavor (2 g), and deionized water (166 g) were added to the solution to prepare a drink for preventing muscle atrophy of the present invention. Ten 100-mL glass bottles were filled with the drink (100 mL), which were then sterilized at 95°C for 15 seconds and sealed. The drink for preventing muscle atrophy contained 50 mg of the milk-derived basic protein fraction of Example product 2 per 100 mL of the drink.

### [Example 8]

### (Preparation of dog food for preventing muscle atrophy)

The decomposed product of the milk-derived basic protein fraction of Example Product 4 (1 kg) was dissolved in deionized water (99 kg). The solution was heated to 50 °C and then mixed with stirring with a T.K. HOMO MIXER (MARK II 160 model; available from Tokushu Kika Kogyo Co., Ltd.) at 3,600 rpm for 40 minutes to prepare a solution containing the decomposed product of the milk-derived basic protein fraction of Example Product 4 (1 g/100 g). Subsequently, the solution (10 kg) of the decomposed product of the milk-derived basic protein fraction was mixed with soybean meal (12 kg), skimmed milk powder (14 kg), soybean oil (4 kg), corn oil (2 kg), palm oil (23.2 kg), corn starch (14 kg), flour (9 kg), bran (2 kg), a vitamin mixture (5 kg), cellulose (2.8 kg), and a mineral mixture (2 kg). The mixture was then sterilized at 120°C for four minutes to produce 100 kg of a dog food for preventing muscle atrophy of the present invention. The dog food for preventing muscle atrophy contained 100 mg of the decomposed product of the milk-derived basic protein fraction of Example Product 4 per 100 g of the dog food.

## Claims

1. A muscle atrophy-preventing agent comprising: a milk-derived basic protein fraction and/or a decomposed product of the milk-derived basic protein fraction as an active ingredient.

2. The muscle atrophy-preventing agent according to claim 1, wherein the decomposed product of the milk-derived basic protein fraction is prepared by treating the milk-derived basic protein fraction with a protease.

3. The muscle atrophy-preventing agent according to claim 2, wherein the protease is at least one selected from the group consisting of pepsin, trypsin, chymotrypsin, pancreatin, and papain.

4. The muscle atrophy-preventing agent according to any one of claims (1) to (3), wherein the milk-derived basic protein fraction has an amino acid composition containing at least 15 weight% basic amino acids.

5. The muscle atrophy-preventing agent according to any one of claims (1) to (3), wherein the milk-derived basic protein fraction is prepared by bringing milk or a milk-derived raw material into contact with a cation exchange resin to adsorb basic proteins on the resin, and eluting a fraction of the adsorbed basic proteins with an eluent having a salt concentration of 0.1 M to 1.0 M.

6. A food or drink for preventing muscle atrophy, a nutritional composition for preventing muscle atrophy, or a feed for preventing muscle atrophy, containing the muscle atrophy-preventing agent according to any one of claims (1) to (4).

7. A method of preventing muscle atrophy, comprising: ingestion of a milk-derived basic protein fraction and/or a decomposed product of the milk-derived basic protein fraction in an amount of 5 mg or more per day.

8. Use of a muscle atrophy-preventing agent comprising a milk-derived basic protein fraction and/or a decomposed product of the milk-derived basic protein fraction as an active ingredient for patients with muscle diseases.

9. Use of a milk-derived basic protein fraction and/or a decomposed product of the milk-derived basic protein fraction in production of a muscle atrophy-preventing agent.
